# EUROPÄISCHE PATENTANMELDUNG

(11) **EP 0 614 889 A1**
(43) Veröffentlichungstag der Anmeldung: **14.09.1994**
(21) Anmeldenummer: 94103635.2
(22) Anmeldetag: 09.03.1994
(51) Int. Cl.: C07D 233/68

(54) **Verfahren zur Herstellung von gegebenenfalls 2-substituierten 5-Chlorimidazol-4-carbaldehyden**

(30) Priorität: 12.03.1993 CH 749/93
(71) Anmelder: LONZA AG, CH-4002 Basel (CH)
(72) Erfinder: Griffiths, Gareth, Dr., Visp (Kanton Wallis) (CH); Imwinkelried, René, Dr., Brig-Glis (Kanton Wallis) (CH); Gosteli, Jacques, Dr., Basel (CH)
(74) Vertreter: Weinhold, Peter, Dr.

(57) **Zusammenfassung**

Es wird ein neues Verfahren zur Herstellung von gegebenenfalls 2-substituierten 5-Chlorimidazol-4-carbaldehyden der allgemeinen Formel 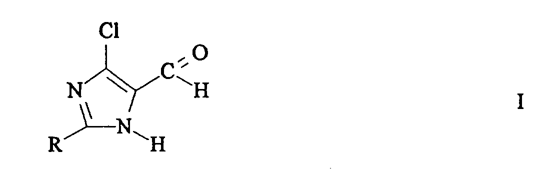
beschrieben.

Diese Verbindungen bilden wertvolle Zwischenprodukte zur Herstellung von blutdrucksenkenden Pharmazeutika oder von herbizidwirksamen Verbindungen.

## Beschreibung

Die Erfindung beinhaltet ein neues Verfahren zur Herstellung von gegebenenfalls 2-substituierten 5-Chlorimidazol-4-carbaldehyden der allgemeinen Formel 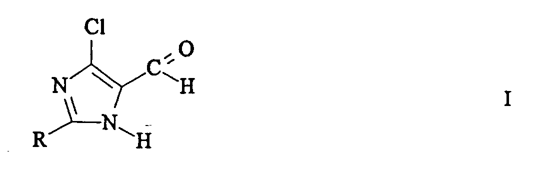
worin R Wasserstoff, eine Alkylgruppe, eine Alkenylgruppe, eine Cycloalkylgruppe, eine Benzylgruppe,eine Phenylgruppe oder eine Arylgruppe bedeutet.

Die gegebenenfalls 2-substituierten 5-Chlorimidazol-4-carbaldehyde der allgemeinen Formel I sind wichtige Ausgangsprodukte zur Herstelung von blutdrucksenkenden Pharmazeutika (US-PS 4355040) oder von herbizidwirksamen Verbindungen (DE-OS 2804435).

Es sind mehrere Wege zur Herstellung der genannten Verbindungen gemäss allg. Formel I bekannt.

So beschreibt die US-PS 4 355 040 ein Verfahren, nach welchem 2-Amino-3,3-dichloracrylnitril mit einem Aldehyd zum entsprechenden Azomethinzwischenprodukt und weiter mit einem Halogenwasserstoff und Wasser zum 2-substituierten 5-Halogen-imidazol-4-carbaldehyd umgesetzt wird. Experimentielle Angaben fehlen in der genannten Patentschrift. Ein grosser Nachteil der Synthese besteht darin, dass das eingesetzte 2-Amino-3,3-dichloracrylnitril zunächst ausgehend von Dichloracetonitril durch dessen Umsetzung mit Blausäue/Natriumcyanid hergestellt werden muss. Die äusserst toxischen Reaktionsteilnehmer und die damit verbundenen sicherheitstechnischen Massnahmen, die bereits für die Bereitstellung des Ausgangsproduktes notwendig sind, machen das Gesamtverfahren industriell untauglich.

Die US-PS 4 355 040 offenbart in einer weiteren Variante ein 3-Stufen Verfahren, worin in einer ersten Stufe ein Amidin-hydrochlorid mit Dihydroxyaceton mit hohem NH₃-Druck ringgeschlossen wird, der Imidazolalkohol halogeniert wird und schliesslich zum Aldehyd oxidiert wird.

Es hat sich gezeigt, dass für die Ringschlussreaktion Drucke von über 20 bar notwendig sind.

Die Oxidation des Alkohols funktioniert gemäss US-PS 4 355 040 in Gegenwart von Chromoxid.

Es ist offensichtlich, dass eine Oxidation mit Schwermetalloxiden, die in der Regel nicht rezirkulierbar sind, nach heutigen ökologischen Gesichtspunkten nicht mehr verantwortbar ist.

Es bestand folglich die Aufgabe, ein Verfahren zu entwickeln, das die genannten Nachteile nicht aufweist.

Die Aufgabe konnte gelöst werden mit einem neuen Verfahren nach Anspruch 1.

Im ersten Schritt wird ein Glycinesterhydrohalogenid der allgemeinen Formel 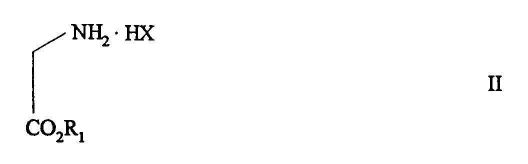
worin R₁ eine Akylgruppe bedeutet und X für ein Halogenatom steht mit einem Imidsäureester der allgemeinen Formel 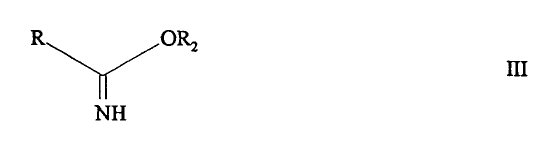
worin R die genannte Bedeutung hat und R₂ eine Alkylgruppe bedeutet in Gegenwart einer Base zum gegebenenfalls 2-substituierten 3,5-Dihydroimidazolin-4-on der allgemeinen Formel 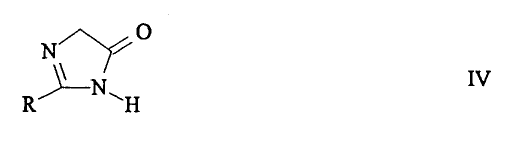
worin R die genannte Bedeutung hat, umgesetst.
Die allgemeinen Bezeichnungen der Gruppen in den Substituenten R, R₁ und R₂ haben nachfolgende Bedeutung.
Unter einer Alkylgruppe werden geradkettige oder verzweigte C₁-C₆-Alkylgruppen, wie z.B. Methyl, Ethyl-, Propyl-, Isopropyl-, n-Butyl, sec. Butyl-, tert. Butyl-, Pentyl- oder Hexylgruppen verstanden. Bevorzugte Alkylgruppe für R ist die n-Propylgruppe oder die n-Butylgruppe. Unter einer Alkenylgruppe wird eine geradekettige oder verzweigte C₁-C₆-Alkenylgruppe, wie z.B. 1-Propenyl, 2-Propenyl, 1-Butenyl, 2-Butenyl, 3-Butenyl, Pentenyl und seine Isomeren oder Hexenyl und seine Isomeren verstanden. Bevorzugte Alkenylgruppe für R ist die 2- oder 3-Butenylgruppe.
Als Vertreter von Cycloalkylgruppen seien die Cyclopropyl-, die Cyclobutyl-, die Cyclopentyl- oder die Cyclohexylgruppe genannt.
Sowohl die Benzyl- als auch die Phenylgruppe kann Substituenten wie die genannten Alkylgruppen, Halogenatome, Nitrogruppen oder Aminogruppen enthalten. Unter der Bezeichnung Halogen wird zweckmässig Chlor, Brom oder Jod, vorzugsweise Chlor verstanden.

Zweckmässig wird so vorgegangen, dass das Glycinesterhydrohalogenid der allg. Formel II in Gegenwart einer Base, zweckmässig bei einem pH-Wert von 7 bis 12, vorzugsweise von 9 bis 11,mit dem Irnidsäureester der allg. Formel III reagiert wird.
Die Glycinesterhydrohalogenide der allgemeinen Formel II sind im Handel erhältliche stabile Verbindungen.
Geeignete Basen sind die Alkalihydroxide, wie z.B. Natriumhydroxid oder Kaliumhydroxid, oder Alkalialkoholate,wie z.B. Natrium- oder Kalium-methylat, -ethylat oder -tert.butylat.

Vorteilhaft liegt die Base in einem geeigneten Lösungsmittel gelöst vor. Besonders geeignet sind aliphatische Alkohole, wie Methanol oder Ethanol. Der Imidsäureester wird zweckmässig ebenfalls in Form einer Lösung in einem inerten Lösungsmittel zugegeben. In der Regel eignen sich hierzu aromatische Lösungsmittel, wie z.B. Toluol oder Chlorbenzol oder die genannten aliphatischen Alkohole besonders gut.

Von Vorteil erfolgt die Umsetzung der Reaktionsteilnehmer Glycinesterhydrohalogenid, Imidsäureester und Base im stöchiometrischen Verhältnis 1:1:1.

Die Umsetzungstemperatur rangiert zweckmässig im Bereich von -20^{o} C bis 50^{o} C, vorzugsweise bei 0^{o} bis 25^{o} C.

Nach einer Umsetzungszeit von wenigen Stunden kann das entsprechende 3,5-Dihydroimidazolin-4-on der allgemeinen Formel IV auf fachmännische Weise, in der Regel durch einfache Filtration, in Ausbeuten von grösser als 95% isoliert werden.

Von Vorteil wird das resultierende Reaktionsgemisch ohne Isolierung des gegebenenfalls 2-substituierten 3,5-Dihydroimidazolin-4-ons für die Weiterumsetzung zum 5-Chlorimidazol-4-carbaldehyd bereitgestellt (Eintopfverfahren).

Die im Rahmen dieser ersten Stufe resultierenden Verbindungen der allg. Formel IV, worin R n-Propyl, n-Butyl, 2- oder 3-Butenyl bedeutet, sind besonders bevorzugt.

Diese Verbindungen sind nicht literaturbekannt und daher ebenfalls Bestandteil der Erfindung.

Diese erste Stufe des erfindungsgemässen Verfahrens beinhaltet eine sprunghafte Verbesserung des bekannten Verfahrens nach R. Jacquier et al. Bull. Soc. Chim. France, 1971, 1040, welches die Umsetzung eines freien Glycinesters mit einem Imidsäureethylester der allg. Formel III (R beschränk: sich auf Methyl, Phenyl, Benzyl) in Abwesenheit eines Lösungsmittels zum entsprechenden 3,5-Dihydroimidazol-4-on umfasst. Nachteilig bei diesem bekannten Verfahren ist, dass der freie Glycinester sehr instabil ist und daher für jede Umsetzung jeweils neu synthetisiert und isoliert werden muss. Gemäss dem bekannten Verfahren konnten nach einer Umsetzungszeit von 24 Std. und langer Ausbeuten von lediglich 30% bis 48% erhalten werden.

Die Umsetzung zum gewünschten gegebenenfalls 2-substituierten 5-Chlorimidazol-4-carbaldehyd der allgemeinen Formel I erfolgt erfindungsgemäss mit Phosphoroxychlorid in Gegenwart von N,N-Dimethylformamid.

Zweckmässig liegt das Molverhältnis der Reaktanden gegebenenfalls 2-substituiertes 3,5-Dihydroimidazolin-4-on zu Phosphoroxychlorid zu N,N-Dimethylformamid im Bereich zwischen 1:1:1 und 1:5:5, bevorzugt bei ungefähr 1:3:3.

Die Umsetzungstemperatur liegt zweckmässig zwischen 50^{o} und 130^{o} C.

Gegebenenfalls kann in Gegenwart eines zusätzlichen inerten Lösungsmittels gearbeitet werden.

Die Isolierung des resultierenden gegebenenfalls 2-substituierten 5-Chlorimidazol-4-carbaldehyds aus dem Reaktionsgemisch erfolgt auf fachmännische Weise vorteilhaft durch dessen Extraktion mit einem geeigneten Lösungsmittel.

### Beispiele

### Beispiel 1

### Herstellung von 2-n-Butyl-3,5-dihydroimidazol-4-on

Zu einer Lösung von Natriumhydroxid 10,1 g (0,25 mol) in Methanol bei 0°C wurden 31,71 g (0,25 mol) Glycinmethylesterhydrochlorid zugegeben. Nach 15 Minuten wurden zur weissen Suspension 126,5 g einer 22,8%igen Lösung von Pentanimidsäuremethylester in Chlorbenzol während 5 Minuten zugetropft. Die hellgelbe Suspension wurde 4 Std. bei Raumtemperatur gerührt und mit Chlorbenzol (100 ml) verdünnt. Das Methanol wurde bei einer Temperatur von 26°C und einem Druck von 30 - 50 mbar abdestilliert, und die orange Suspension wurde mit Methylenchlorid (100 ml) verdünnt und anschliessend filtriert. Nach Entfernung des Lösungsmittels vom Filtrat wurden 34,09g (97%) der Titelverbindung (Gehalt >95% nach GC und ¹H-NMR) erhalten.

- ¹H-NMR (CDCl₃, 300 Mhz) δ in ppm: 0,95 (t, 3H); 1,45 (m, 2H);
1,68 (m, 2H);
2,48 (t, 2H);
4,1 (m, 2H);
9,3 (br. s, 1H).

### Beispiel 2

### Herstellung von 2-n-Butyl-5-chlorimidazol-4-carbaldehyd

Zu einer Lösung von Phosphoroxychlorid (26,83 g, 175 mmol) in Chlorbenzol (50 ml) bei Raumtemperatur wurde 2-n-Butyl-2-imidazolin-5-on (9,81 g, 70 mmol) zugegeben. Die orange Suspension wurde innerhalb von 5 Minuten auf 100°C erhitzt, danach wurde N,N-Dimethylformamid (12,79g 175 mmol) innerhalb von 3 Minuten zugegeben. Das schwarze Gemisch wurde 2 Std. bei 100°C gehalten, auf 40°C gekühlt und auf Wasser (84 ml) gegossen. Nach Zugabe von Essigester (42 ml) wurde das Gemisch 15 Minuten bei 26° - 28°C gerührt und anschliessend durch Zugabe von 30%-iger Natronlauge (67 ml) auf pH 7 gestellt. Die Phasen wurden getrennt, und die wässrige Phase wurde zweimal mit ie 70 ml Essigester extrahiert. Die vereinigten organischen Phasen wurden getrocknet (MgSO₄) filtriert und am Rotavapor eingeengt. Die Titelverbindung, wurde in einer Ausbeute von 8,31 g (64%) bezogen auf 2-n-Butyl-2-imidazolin-5-on erhalten.

### Beispiel 3

### Herstellung von 2-n-Butyl-5-chlorimidazol-4-carbaldehyd (Verfahren ohne Isolation von 2-n-Butyl-2-imidazolin-5-on)

Zu einer auf 0°C gekühlten Lösung von 10,17 g (0,25 Mol) Natriumhydroxid in Methanol (80 ml) wurden 31,71 g (0,25 Mol) Glycinmethylesterhydrochlorid als Feststoff in einer Portion zugegeben, wobei sich die weisse Suspension (NaCl fällt aus) auf -8°C abkühlte. Die Temperatur stieg innert 15 Minuten auf O°C, danach wurden 127,0 g einer 22,68%igen Lösung von Pentanimidsäuremethylester in Chlorbenzol (0,25 Mol Pentanimidsäuremethylester) wahrend 15 Minuten zugegeben. Die Temperatur wurde innerhalb von 1 Std. auf 21°C steigengelassen, anschliessend wurde die gelbbraune Suspension 3,5 Std. bei 21°C gerührt. Nach Zugabe von Chlorbenzol (400 ml) wurden ca. 240 g von einem Gemisch aus Methanol, Wasser und Chlorbenzol bei 30°C abdestilliert. Zur zurückbleibenden Suspension (ca. 420 g) wurde Phosphoroxychlorid (107,33 g, 0,7 Mol) zugegeben (Temperatur stieg auf 35°C). Das trübe, orange Reaktionsgemisch wurde auf 100°C erhitzt, danach wurde N,N-Dimethylformamid (51,71 g, 0,70 Mol) während 5 Minuten zugetropft (die Temperatur stieg auf 108°C). Nach 2 Std. bei 100°C wurde das schwarze Gemisch auf 75°C abgekühlt und in 300 g auf 10°C gekühltes Wasser gegossen. Das Gemisch wurde mit Essigester (150 ml) verdünnt, 15 Minuten bei 50°C gerührt und durch Zugabe von 160 ml 30%iger Natronlauge auf pH 1 gestellt. Die Phasen wurden getrennt, und die wässrige Phase wurde zweimal mit je 250 ml Essigester extrahiert. Die vereinigten organischen Phasen wurden getrocknet (MgSO₄), filtriert und am Rotavapor eingeengt. Die Titelverbindung wurde in einer Ausbeute von 34,3 g (73%), bezogen auf das Glycinmethylesterhydrochlorid, erhalten.

### Beispiel 4

### Herstellung von 2-n-Butyl-5-chlorimidazol-4-carbaldehyd (Verfahren ohne Isolation von 2-n-Butyl-2-imidazolin-5-on)

Zu einer auf 0°C gekühlten Lösung von 10,15 g (0,25 Mol) Natriumhydroxid in Methanol (80 ml) wurden 31,72 g (0,25 Mol) Glycinmethylesterhydrochlorid als Feststoff in einer Portion zugegeben, wobei sich die weisse Suspension (NaCl fällt aus) auf -9°C abkühlte. Die Temperatur stieg innert 15 Minuten auf 0°C, danach wurden 127,0 g einer 22,68%igen Lösung von Pentanimidsäuremethylester in Chlorbenzol (0,25 Mol Pentanimidsäuremethylester) während 15 Minuten zugegeben. Die Temperatur wurde innerhalb von 1 Std. auf 21°C steigengelassen, anschliessend wurde die gelbbraune Suspension 3,5 Std. bei 21°C gerührt. Nach Zugabe von Chlorbenzol (200 ml) wurde ca. 210 g von einem Gemisch aus Methanol, Wasser und Chlorbenzol bei 30°C abdestilliert. Zur zurückbleibenden Suspension (ca. 226 g) wurde Phosphoroxychlorid (107,33 g, 0,70 Mol) zugegeben (Temperatur stieg auf 35°C). Das trübe, orange Reaktionsgemisch wurde auf 100°C erhitzt, danach wurde DMF (51,71 g, 0,70 Mol) während 5 Minuten zugetropft (die Temperatur stieg auf 108°C). Nach 2 Std. bei 100°C wurde das schwarze Gemisch auf 40°C abgekühlt und in 300 g auf 10°C gekühltes Wasser gegossen. Das Gemisch wurde mit Essigester (150 ml) verdünnt, 15 Minuten bei 50°C und durch Zugabe von 160 mi 30%iger Natronlauge auf pH 1 gestellt. Die Phasen wurden getrennt, und die wässrige Phase wurde zweimal mit je 250 ml Essigester extrahiert. Die vereinigten organischen Phasen wurden getrocknet (MgSO₄), filtriert, am Rotavapor auf 125 g eingeengt und auf-10°C gekühlt. Das ausgefallene Produkt wurde abfiltriert, mit kaltem Essigester gewaschen und bei 50°C getrocknet. Die Ausbeute betrug 19,01 g (40% bez. auf Glycinmethylesterhydrochlorid). Die Mutterlauge enthielt nach GC mit int. Std. weitere 11,66 g der Titelverbindung, somit betrug die Gesamtausbbeute 65% bez. auf Glycinmethylesterhydrochlorid.

### Beispiel 5

### Herstellung von 2-n-Propyl-3,5-dihydroimidazol-4-on

Zu einer Lösung von Natriumhydroxid (5,56g, 139 mmol) in Methanol (55ml) bei 0°C wurden 17,39 g (138 mmol) Glycinmethylesterhydrochlorid zugegeben. Nach 15 Minuten wurden zur weissen Suspension während 8 Minuten 14,50 g (Gehalt 96,3%, 138 mmol) Butanimidsäuremethylester zugetropft. Das Gemisch wurde 3 Std. bei Raumtemperatur gerührt und danach am Rotavapor eingeengt. Der Rückstand wurde mit CH₂Cl₂ (250 ml) versetzt, und die entstandene Suspension wurde filtriert. Das Filtrat wurde am Rotavapor eingeengt, nochmals mit CH₂Cl₂ (250 ml) versetzt und wieder filtriert. Nach Entfernung des Lösungsmittels wurde die Titelverbindung erhalten. (15,13 g, Gehalt > 95% nach ¹H-NMR, 83% Ausbeute)

- ¹H-NMR (CDCl₃, 400 Mhz) δ in ppm: 1,03 (t, 3H); 1,75 (m, 2H);
2,46 (t, 2H);
4,12 (s, 2H);
9,98 (br. s, 1H).

### Beispiel 6

### Herstellung von 2-n-Propyl-5-chlorimidazol-4-carbaldehyd

Zu einem Gemisch von 2-n-Propyl-3,5-dihydroimidazol-4-on (4,49 g, 35,6 mmol) und POCl₃ (14,76 g, 96,3 mmol) in Chlorbenzol (40 ml) bei 100°C wurde N,N-Dimethylformamid (7,04 g, 96,3 mmol) zugegeben. Das Gemisch wurde 2 Std. bei 100°C erhitzt, gekühlt und auf 40 g Eis gegossen. Das Gemisch wurde durch Zugabe von 30%iger Natronlauge (22,5 ml) auf pH 1 gestellt und die Phasen wurden getrennt. Die wässrige Phase wurde zweimal mit je 40 ml Essigester extrahiert, und die vereinigten organischen Phasen wurden mit Wasser (20 ml) gewaschen und über Kieselgel filtriert. Nach Entfernung des Lösungsmittels vom Filtrat wurden 2,79 g eines hellbraunen Feststoffes erhalten. Umkristallisation aus Essigester/Petrolether lieferte die Titelverbindung (2,04 g, 33%).
Smp.: 133,3° - 137,5°C

- ¹H-NMR (CDCl₃, 400 Mhz) δ in ppm: 1,01 (t, 3H); 1,84 (m, 2H);
2,83 (t, 2H);
9,64 (s, 1H);
11,56 (br. s, 1H).

### Beispiel 7

### Herstellung von 2-But-2-enyl-3,5-dihydroimidazol-4-on

Entsprechend Beispiel 1 wurde durch Umsetzung von Glycinmethylesterhydrochlorid und 3-Pentenimidsäuremethylester das Titelprodukt hergestellt.

- ¹H-NMR (CDCl₃, 400 Mhz:) δ in ppm: 1,75 (d, 3H); 3,19 (d, 2H);
4,12 (s, 2H);
5,54 (m, 1H);
5,75 (m, 1H);
9,20 (br. s, 1H).

## Patentansprüche

1. Verfahren zur Herstellung von gegebenenfalls 2-substituierten 5-Chlorimidazol-4-carbaldehyden der allgemeinen Formel 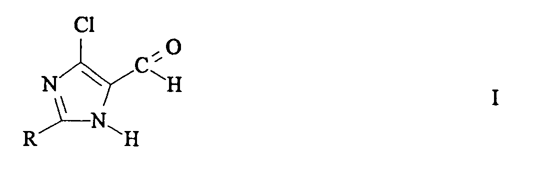 worin R Wasserstoff, eine Alkylgruppe, eine Alkenylgruppe, eine Cyclo-alkylgruppe, eine Benzylgruppe, eine Phenylgruppe oder eine Arylgruppe bedeutet,dadurch gekennzeichnet, dass in der ersten Stufe ein Glycinesterhydrohalogenid der allg. Formel 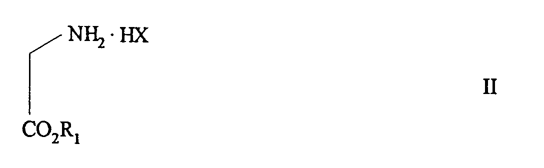 worin R₁ eine Alkylgruppe und X ein Halogenatom bedeutet mit einem Imidsäureester der allgemeinen Formel 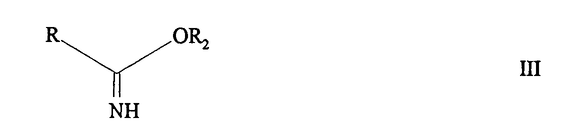 worin R die genannte Bedeutung hat, und R₂ eine Alkylgruppe bedeutet in Gegenwart einer Base zum gegebenenfalls 2-substituierten 3,5-Dihydroimidazolin-4-on der allgemeinen Formel 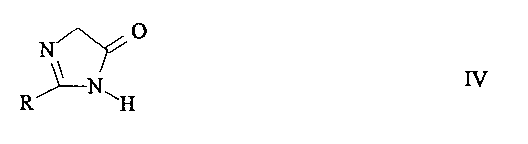 worin R die genannte Bedeutung hat, umgesetzt wird und weiter mit Phosphoroxychlorid in Gegenwart von N,N-Dimethylformamid zum Endprodukt umgesetzt wird.

2. Verfahren nach Patentanspruch 1, dadurch gekennzeichnet, dass als Base in der ersten Stufe ein Alkalihydroxid oder ein Alkalialkoholat verwendet wird.

3. Verfahren nach Patentansprüchen 1 oder 2, dadurch gekennzeichnet, dass die Umsetzung in der ersten Stufe in einem pH-Bereich zwischen 7 und 12 durchgeführt wird.

4. Verfahren nach einem der Patentansprüche 1-3, dadurch gekennzeichnet, dass die Reaktionsteilnehmer der ersten Stufe Glycinesterhydrohalogenid, Imidsäureester und Base im stöchiometrischen Molverhältnis 1:1:1 umgesetzt werden.

5. Verfahren nach einem der Patentansprüche 1-4, dadurch gekennzeichnet, dass die Umsetzungstemperatur in der ersten Stufe zwischen -20°C und 50°C liegt.

6. Verfahren nach einem der Patentansprüche 1-5, dadurch gekennzeichnet, dass die Reaktionsteilnehmer der zweiten Stufe gegebenenfalls 2-substituiertes 3,5-Dihydroimidazolin-4-on, Phosphoroxychlorid, N,N-Dimethylformamid im Molverhältnis 1:1:1 bis 1:5:5 umgesetzt werden.

7. Verfahren nach einem der Patentansprüche 1-6, dadurch gekennzeichnet, dass die Umsetzungstemperatur in der zweiten Stufe zwischen 50°C und 130°C liegt.

8. Verfahren nach Patentanspruch 1, dadurch gekennzeichnet, dass das gegebenenfalls 2-substituierte 3,5-Dihydroimidazolin-4-on der allgemeinen Formel IV im Verfahrensverlauf nicht isoliert wird.

9. Gegebenenfalls 2-substituiertes 3,5-Dihydroimidazolin-4-one der allgemeinen Formel IV, worin R n-Propyl, n-Butyl, 2-Butenyl oder 3-Butenyl bedeutet.
